(19) Europäisches Patentamt
European Patent Office
Office européen des brevets

(11) Publication number: 0 018 683
B1

(12) EUROPEAN PATENT SPECIFICATION

(45) Date of publication of patent specification: 22.12.82

(51) Int. Cl.[3]: C 07 C 15/00, C 07 C 1/04, B 01 J 23/86

(21) Application number: 80200354.1

(22) Date of filing: 17.04.80

# (54) Process for the preparation of hydrocarbons, and hydrocarbons so prepared.

(30) Priority: 25.04.79 NL 7903243

(43) Date of publication of application:
12.11.80 Bulletin 80/23

(45) Publication of the grant of the patent:
22.12.82 Bulletin 82/51

(84) Designated Contracting States:
BE DE FR GB IT

(56) References cited:
NL - A - 7 613 457
NL - A - 7 708 511
NL - A - 7 803 707
NL A - 7 811 821
NL - A - 7 812 292
US - A - 2 497 964
US - A - 2 540 581
US - A - 2 585 981

(73) Proprietor: SHELL INTERNATIONALE RESEARCH MAATSCHAPPIJ B.V.
Carel van Bylandtlaan 30
NL-2596 HR Den Haag (NL)

(72) Inventor: Schaper, Lambert
Badhuisweg 3
NL-1031 CM Amsterdam (NL)
Inventor: Sie, Swan Tiong
Badhuisweg 3
NL-1031 CM Amsterdam (NL)

(74) Representative: Puister, Antonius Tonnis, Mr. et al,
P.O. Box 302
NL-2501 CH The Hague (NL)

EP 0 018 683 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the preparation of hydrocarbons, and hydrocarbons so prepared

The invention relates to a process for the preparation of an aromatic hydrocarbon mixture from a mixture of carbon monoxide and hydrogen using a mixture of two catalysts of which one has the capability of catalysing the conversion of an $H_2/CO$ mixture into acyclic oxygen-containing hydrocarbons and the other is a crystalline silicate which has the capability of catalysing the conversion of acyclic oxygen-containing hydrocarbons into aromatic hydrocarbons. The said crystalline silicates are characterized in having the following properties after 1 hour's calcining in air at 500°C:
(a) thermally stable up to a temperature above 600°C,
(b) an X-ray powder diffraction pattern showing, inter alia, the reflections given in Table A,

TABLE A

| Radiation: Cu-K$\alpha$ 2 $\theta$ | Wavelength 0.15418 nm relative intensity |
|---|---|
| 7.8—8.2 | S |
| 8.7—9.1 | M |
| 11.8—12.1 | W |
| 12.4—12.7 | W |
| 14.6—14.9 | W |
| 15.4—15.7 | W |
| 15.8—16.1 | W |
| 17.6—17.9 | W |
| 19.2—19.5 | W |
| 20.2—20.6 | W |
| 20.7—21.1 | W |
| 23.1—23.4 | VS |
| 23.8—24.1 | VS |
| 24.2—24.8 | S |
| 29.7—30.1 | M |

wherein the letters used have the following meanings:
VS=very strong; S=strong; M=moderate; W=weak; $\theta$=angle according to Bragg;
(c) after conversion of the silicate into the H-form and after evacuation at $2\times10^{-9}$ bar and 400°C for 16 hours and measured at a hydrocarbon pressure of $8\times10^{-2}$ bar and 100°C, the adsorption of n-hexane is at least 0.8 mmole/g, the adsorption of 2,2-dimethylbutane at least 0.5 mmole/g, and the ratio

$$\frac{\text{adsorption of n-hexane}}{\text{adsorption of 2,2-dimethylbutane}} \text{ at least } 1.5;$$

(d) the composition, expressed in moles of the oxides, is as follows:

$$y(1.0\pm0.3)M_{2/n}O \cdot y(a\ Fe_2O_3 \cdot b\ Al_2O_3) \cdot SiO_2,$$

wherein M=H and alkali metal or alkaline-earth metal; n=the valency of M; $0<y\leqslant0.1$, $a\geqslant0$; $b\geqslant0$, and $a+b=1$.

For the adsorption measurements mentioned under (c) the silicate should first be converted into the H-form. This conversion is effected by boiling the silicate calcined at 500°C with 1.0 molar $NH_4NO_3$ solution, washing with water, boiling again with 1.0 molar $NH_4NO_3$ solution and washing, drying at 120°C and calcining at 500°C. Such a process is known from Netherlands patent application No. 7708511 which relates to an improved process for preparing aromatic hydrocarbon mixtures from acyclic oxygen-containing hydrocarbons according to the Netherlands patent application No. 7613957, in which an oxygen-containing hydrocarbon or a mixture of hydrocarbons consisting substantially of oxygen-containing hydrocarbons is contacted with a catalyst containing a crystalline silicate which
a) is thermally stable up to temperatures above 600°C;
b) is able—after dehydration at 400°C in vacuum—to adsorb more than 3%w water at 25°C and saturated water vapour pressure, and
c) in dehydrated form, has the following overall composition, expressed in moles of the oxides

$$(1.0\pm0.3)\ (R)_{2/n}O \cdot [a\ Fe_2O_3 \cdot b\ Al_2O_3] \cdot (10\text{—}600)SiO_2,$$

where R=one or more mono- or bivalent cations,

$a \geqslant 0.1$,
$b \geqslant 0$,
$a+b=1$, and
n is the valency of R.

The improvement according to Netherlands patent application No. 7708511 consists in that a mixture of carbon monoxide and hydrogen is converted in one step into an aromatic hydrocarbon mixture by contacting the gas mixture with a bifunctional catalyst containing, in addition to the crystalline silicate, one or more metal components having activity for the conversion of an $H_2/CO$ mixture into oxygen-containing hydrocarbons.

The silicate catalyst used in the process according to Netherlands patent application No. 7708511 is described in more detail in Netherlands patent application No. 7613957.

In an investigation by the Applicant concerning the use of the process for the preparation of aromatic hydrocarbon mixtures, which was started from $H_2/CO$ mixtures with an $H_2/CO$ molar ratio between 0.25 and 0.75, the stability of the catalyst mixture was found to be unsatisfactory.

Further investigation by the Applicant concerning this subject showed that addition of water to the $H_2/CO$ mixture in an amount of at least 2.5 mole%, based on the $H_2/CO$ mixture, may yield a considerable improvement of the stability of the catalyst mixture. It has further been found that addition of water to these low-hydrogen $H_2/CO$ mixtures in an amount which does not exceed a certain value, may yield a considerable improvement of the conversion of these $H_2/CO$ mixtures. The maximum amount of water that can be added to the $H_2/CO$ mixture with a view to improving the conversion of the $H_2/CO$ mixture at a given temperature, pressure, space velocity and catalyst mixture is dependent on the $H_2/CO$ molar ratio of the feed and on the consumption ratio of the $H_2/CO$ mixture when the process is carried out under the conditions given, without water addition. The maximum amount of water in mole% based on the $H_2/CO$ mixture, is given by the formula:

$$\frac{3(V-R)}{(1+R)(1+V)},$$

wherein V=the consumption ratio of the $H_2/CO$ mixture when the process is carried out without water addition, and R=the $H_2/CO$ molar ratio of the feed.

The present patent application therefore relates to a process for the preparation of an aromatic hydrocarbon mixture in which a mixture of carbon monoxide and hydrogen with an $H_2/CO$ molar ratio between 0.25 and 0.75 is contacted with a mixture of two catalysts of which one is capable of catalyzing the conversion of an $H_2/CO$ mixture into acyclic oxygen-containing hydrocarbons and the other is a crystalline silicate as defined above, and in which to the $H_2/CO$ mixture an amount of water is added which—in mole% based on the $H_2/CO$ mixture—is at least 2.5 and at most

$$\frac{3(V-R)}{(1+R)(1+V)}.$$

For determining the maximum amount of water that can be added at a given temperature, pressure, space velocity and catalyst mixture to an $H_2/CO$ mixture with a given R between 0.25 and 0.75, in order to obtain an improved stability of the catalyst mixture and an improved conversion of the $H_2/CO$ mixture, V should be known. This parameter can be found in a simple way with the aid of an experiment carried out under the conditions given, but without water addition, V is calculated with the formula:

$$V=\frac{C_{H_2}}{C_{CO}}\times R,$$

wherein $C_{H_2}$ and $C_{CO}$ are the initial conversions found in the experiment for $H_2$ and CO, respectively.

With regard to the amount of water that can be added to the $H_2CO$ mixture the following remark should be made. If in the experiment, under given conditions without water addition, for V a value is found such that the formula

$$\frac{3(V-R)}{(1+R)(1+V)}$$

yields a value smaller than 2.5, this means that it is not possible under the given conditions to reach an

improvement of the conversion of the $H_2/CO$ mixture by adding water in an amount of more than 2.5 mole%, based on the $H_2/CO$ mixture. The invention does not hold for situations in which the formula

$$\frac{3(V-R)}{(1+R)(1+V)}$$

yields a value lower than 2.5. These situations are therefore outside the scope of the present patent application.

In the process according to the invention an amount of water should be added to the $H_2/CO$ mixture which—in mole%—based on the $H_2/CO$ mixture—is at least 2.5 and at most

$$\frac{3(V-R)}{(1+R)(1+V)}$$

In view of the improvement to be achieved in the conversion of the $H_2/CO$ mixture it is preferred to add an amount of water which—in mole%, based on the $H_2CO$ mixture—lies between

$$\frac{1.1(V-R)}{(1+R)(1+V)} \quad \text{and} \quad \frac{1.9(V-R)^2}{(1+R)(1+V)}$$

and in particular between

$$\frac{1.3(V-R)}{(1+R)(1+V)} \quad \text{and} \quad \frac{1.7(V-R)}{(1+R)(1+V)}$$

In the process according to the invention the starting material is an $H_2/CO$ mixture with a molar ratio between 0.25 and 0.75. Such a mixture can very suitably be prepared by steam gasification of a carbon-containing material. Examples of such materials are brown coal, anthracite, coke, crude mineral oil and fraction thereof and oils recovered from tar sand and bituminous shale. The steam gasification is preferably carried out at a temperature between 900 and 1500°C and a pressure between 10 and 50 bar.

The process according to the invention is preferably carried out at a temperature of 200—500°C and in particular of 300—450°C, a pressure of 1—150 bar and in particular of 5—100 bar and a space velocity of 50—5000 and in particular of 300—3000 l (NTP) gas/l catalyst/h.

In the process according to the invention a mixture of two catalysts is used, which, for the sake of convenience, will be designated catalysts A and B. Catalyst A is the catalyst which is capable of catalyzing the conversion of an $H_2/CO$ mixture into acyclic oxygen-containing hydrocarbons and catalyst B is the crystalline silicate. Catalysts that are preferably used as A-catalysts are those which are capable of converting an $H_2/CO$ mixture into substantially methanol and/or dimethyl ether. If in the process according to the invention the aim is to prepare a product consisting substantially of hydrocarbons boiling in the gasoline range, a catalyst which contains zinc together with chromium can very suitably be used as the A-catalyst. When used such a catalyst, it is preferred to choose one in which the atomic percentage of zinc, based on the sum of zinc and chromium, is at least 60% and in particular 60—80%. If in the process according to the invention the aim is to prepare, in addition to hydrocarbons boiling in the gasoline range, a fuel gas with a high calorific value, a catalyst which contains zinc together with copper can very suitably be used as the A-catalyst. The catalyst mixture that is used in the process according to the invention may be a macromixture or a micromixture. In the first case the catalyst mixture consists of two kinds of macroparticles, of which one kind consists completely of catalyst A, and the other kind completely of catalyst B. In the second case the catalyst mixture consists of one kind of macroparticles, each macroparticle being built up of a great number of microparticles of each of the catalysts A and B. Catalyst mixtures in the form of micromixtures may be prepared, for instance, by thoroughly mixing a fine powder of catalyst A with a fine powder of catalyst B and shaping the mixture into larger particles, for instance by extruding or tabletting. In the process according to the invention it is preferred to use catalyst mixtures in the form of micromixtures. In view of the activity of the catalyst mixtures, preferred mixtures are those containing per part by volume of catalyst B, 1—5 parts by volume of catalyst A.

The crystalline silicate that is present in the catalyst mixtures as catalyst B, has been defined, inter alia, with reference to the X-ray powder diffraction pattern shown by the silicate after 1 hour's calcining in air at 500°C. This X-ray powder diffraction pattern should contain, inter alia, the reflections shown in Table A. The complete X-ray powder diffraction pattern of a typical example of a silicate eligible for use according to the invention is shown in Table B.

(Radiation: Cu-K$\alpha$; wavelength: 0.15418 nm).

TABLE B

| 2 $\theta$ | Relative intensity (100. $I/I_o$) | Description |
|---|---|---|
| 8.00 | 55 | SP |
| 8.90 | 36 | SP |
| 9.10 | 20 | SR |
| 11.95 | 7 | NL |
| 12.55 | 3 | NL |
| 13.25 | 4 | NL |
| 13.95 | 10 | NL |
| 14.75 | 9 | BD |
| 15.55 | 7 | BD |
| 15.95 | 9 | BD |
| 17.75 | 5 | BD |
| 19.35 | 6 | NL |
| 20.40 | 9 | NL |
| 20.90 | 10 | NL |
| 21.80 | 4 | NL |
| 22.25 | 8 | NL |
| 23.25 | 100* | SP |
| 23.95 | 45 | SP |
| 24.40 | 27 | SP |
| 25.90 | 11 | BD |
| 26.70 | 9 | BD |
| 27.50 | 4 | NL |
| 29.30 | 7 | NL |
| 29.90 | 11 | BD |
| 31.25 | 2 | NL |
| 32.75 | 4 | NL |
| 34.40 | 4 | NL |
| 36.05 | 5 | BD |
| 37.50 | 4 | BD |
| 45.30 | 9 | BD |

* $I_o$=intensity of the strongest separate reflection present in the pattern.

The letters used in Table B for describing the reflections have the following meanings: SP=sharp; SR=shoulder; NL=normal; BD=broad; $\theta$=angle according to Bragg.

The crystalline silicates which are used in the catalyst mixtures can be prepared from an aqueous mixture as the starting material which contains the following compounds: one or more compounds of an alkali metal or alkaline-earth metal (M), one or more compounds containing an organic cation (R) or from which such a cation s formed during the preparation of the silicate, one or more silicon compounds and one or more aluminium and/or iron compounds. The preparation is performed by maintaining the mixture at elevated temperature until the silicate has been formed and subsequently separating the crystals of the silicate from the mother liquor. In the preparation of the silicates it is preferred to start from a base mixture in which M is present in a sodium compound and R in a tetrapropylammonium compound.

The silicates prepared in the way described above contain alkali metal ions and/or alkaline-earth metal ions and organic cations. By using suitable exchange methods the alkali metal ions and alkaline-earth metal ions can be replaced by other cations, such as hydrogen ions or ammonium ions. Organic cations can be very suitably converted into hydrogen ions by calcining the silicates. The crystalline silicates which are used in the catalyst mixtures preferably have an alkali metal content of less than 0.1 %w and in particular less than 0.01 %w. If desired, a binder material such as bentonite or kaolin may be incorporated into the catalyst mixtures.

In view of the stability of the catalyst mixtures, in the process according to the invention preference is given to crystalline silicates with an average crystallite size of less than 3000 nm and in particular less than 1000 nm. The average crystallite size of the silicates can be adjusted with the aid of the molar ratio of $(R)_{2/p}O$ (wherein p represents the valency of R) to $SiO_2$ in the starting mixture, in the sense that silicates with a lower average crystallite size are obtained according as the molar ratio of $(R)_{2/p}O$ to $SiO_2$ in the starting mixture is chosen higher.

If in the process according to the invention the aim is to reach a high $C_5^+$ selectivity, this can be realized in three ways by choosing the crystalline silicate in the catalyst mixture. In the first place a crystalline silicate may be used to this end for which holds that a=1. Further crystalline silicate may be used for which holds that a=0 and $y \leq 0.005$. Finally, a crystalline silicate may be used for

which holds that a=0 and $y \geq 0.005$, which silicate contains, in addition, one or more elements selected from the group formed by manganese, calcium, magnesium and titanium. The value of y in the formula which gives the composition of the silicates can be adjusted with the aid of the molar ratio of $SiO_2$ on the one hand to $Al_2O_3$ and/or $Fe_2O_3$ on the other hand in the starting mixture, in the sense that silicates with a lower value for y are obtained according as the molar ratio of $SiO_2$ on the one hand to $Al_2O_3$ and/or $Fe_2O_3$ on the other hand in the starting mixture is chosen higher.

The process according to the invention can very suitably be carried out by conducting the feed in upward or downward direction through a vertically mounted reactor, in which a fixed or a moving bed of the catalyst mixture concerned is present. The process may, for instance, be carried out by conducting a feed in upward direction through a vertically mounted catalyst bed, using such a gas rate that expansion of the catalyst bed occurs. If desired, the process can also be carried out using a suspension of the catalyst mixture in a hydrocarbon oil. Depending on whether the process is carried out with a fixed catalyst bed, an expanded catalyst bed or a catalyst suspension, preference is given to catalyst particles with a diameter between 1 and 5 mm, 0.5 and 2.5 mm and 20 and 150 m, respectively.

The invention will now be explained with reference to the following example.

Example

A crystalline silicate (silicate A) was prepared as follows:

A mixture of $SiO_2 \cdot Na_2AlO_2 \cdot NaOH$ and $[(C_3H_7)_4N]OH$ in water with the molar composition

$$5\ Na_2O \cdot Al_2O_3 \cdot 22.5[(C_3H_7)_4N]_2O \cdot 125\ SiO_2 \cdot 2250\ H_2O$$

was heated for 48 hours in an autoclave at 150°C under autogenous pressure. After the reaction mixture has cooled down, the silicate formed was filtered off, washed with water until the pH of the wash water was about 8 and dried for two hours at 120°C. After 1 hour's calcining in air at 500°C silicate A had the following properties:

(a) thermally stable up to a temperature above 800°C;

(b) an X-ray powder diffraction pattern substantially equal to the one given in Table B;

(c) after conversion of the silicate into the H-form and after evacuation at $2 \times 10^{-9}$ bar and 400°C for 16 hours and measured at a hydrocarbon pressure of $8 \times 10^{-2}$ bar and 100°C, the adsorption of n-hexane is 1.2 mmol/g, the adsorption of 2,2-dimethylbutane 0.7 mmol/g and the ratio

$$\frac{\text{adsorption of n-hexane}}{\text{adsorption of 2,2-dimethylbutane}}\ 1.7, \text{ and}$$

(d) the composition, expressed in moles of the oxides, is

$$0.011\ M_2O \cdot 0.011\ Al_2O_3 \cdot SiO_2,$$

wherein M=H and Na.

From silicate A, which had an average crystallite size of 280 nm, a silicate B was prepared by boiling the material calcined at 500°C with 1.0 molar $NH_4NO_3$ solution, washing with water, boiling again with 1.0 molar $NH_4NO_3$ solution and washing, drying for 2 hours at 120°C and calcining for 1 hour at 500°C.

A crystalline silicate (silicate C) was prepared in substantially the same way as silicate A, the difference being that for the preparation of silicate C the starting material was an aqueous mixture with the molar composition:

$$1.5\ Na_2O \cdot Al_2O_3 \cdot 2.25[(C_3H_7)_4N]_2O \cdot 37.5\ SiO_2 \cdot 675\ H_2O.$$

After 1 hour's calcining in air at 500°C silicate C was completely equal to silicate A as regards thermal stability, X-ray powder diffraction pattern and adsorption behaviour. The composition of silicate C (after calcining), expressed in moles of the oxides, was as follows:

$$0.025\ M_2O \cdot 0.025\ Al_2O_3 \cdot SiO_2, \text{ wherein M=H and Na.}$$

From silicate C, which had an average crystallite size of 250 nm, a silicate D was prepared in the same way as described above for the preparation of silicate B from silicate A. From silicate D a silicate E was prepared, which contained 3 %w manganese, by impregnating silicate D with an aqueous solution of a manganese salt, followed by drying and calcining of the impregnated material.

Two catalyst mixtures (I and II) were prepared by mixing silicate B with an $Fe_2O_3$—$Cr_2O_3$ composition, and mixing silicate E with a $ZnO$—$Cr_2O_3$ composition, respectively. Catalyst mixture I

contained the silicate B and the $Fe_2O_3$—$Cr_2O_3$ composition in a volume ratio of 1:1. Catalyst mixture II contained the silicate E and the $ZnO$—$Cr_2O_3$ composition in a volume ratio of 1:3. The atomic Zn percentage of the $ZnO$—$Cr_2O_3$ composition, based on the sum of Zn and Cr, was 70%.

Catalyst mixtures I and II were tested for the preparation of an aromatic hydrocarbon mixture from an $H_2/CO$ mixture.

The testing of catalyst mixture I was performed by means of three experiments (1—3), which were carried out in a 50ml reactor in which a fixed catalyst bed with a volume of 7.5 ml was present. An $H_2/CO$ mixture with an $H_2/CO$ molar ratio of 0.5 was conducted over the catalyst at a temperature of 375°C, a pressure of 30 bar and a space velocity of 1000 $l.l^{-1}.h^{-1}$. Experiment 1 was carried out without water addition to the $H_2/CO$ mixture. In experiments 2 and 3 water was added to the $H_2/CO$ mixture, in experiment 2 in an amount of 6.6 mole% and in experiment 3 in an amount of 16.6 mole%. In experiments 1—3 the following results were obtained:

Experiment 1 (no water added)

$$C_{H_2}=79 \text{ mole\%}$$

$$C_{CO}=66 \text{ mole\%}$$

Initial conversion of the $H_2/CO$ mixture: 70 mole%. Conversion of the $H_2/CO$ mixture after 100 h: 67 mole%.

With the use of the values for $C_{H_2}$ and $C_{CO}$ which were determined in this experiment, it can be calculated that for obtaining an improved stability and conversion of the $H_2/CO$ mixture, according to the invention, an amount of water of at most 12.5 mole% should be added.

Experiment 2 (6.6 mole% water added)

Initial conversion of the $H_2/CO$ mixture: 76 mole%. Conversion of the $H_2/CO$ mixture after 100 h: 75 mole%.

Experiment 3 (16.6 mole% water added)

Initial conversion of the $H_2/CO$ mixture: 53 mole%.

The testing of catalyst mixture II was performed by means of two experiments (4 and 5), which were carried out in a 250-ml reactor in which a fixed catalyst bed with a volume of 50 ml was present. An $H_2/CO$ mixture with an $H_2/CO$ molar ratio of 0.45 was conducted over the catalyst at a temperature of 375°C, a pressure of 60 bar and a space velocity of 200 $l.l^{-1}.h^{-1}$. Experiment 4 was carried out without water addition to the $H_2/CO$ mixture. In experiment 5, 6.2 mole% water was added to the $H_2/CO$ mixture. In experiments 4 and 5 the following results were obtained:

Experiment 4 (no water added)

$$C_{H_2}=93 \text{ mole\%}$$

$$C_{CO}=80 \text{ mole\%}$$

Initial conversion of the $H_2/CO$ mixture: 84 mole%. Conversion of the $H_2/CO$ mixture after 600 h: 79 mole%.

With the use of the values for $C_{H_2}$ and $C_{CO}$ which were determined in this experiment, it can be calculated that for obtaining an improved stability and conversion of the $H_2/CO$ mixture, according to the invention, an amount of water of at most 9.5 mole% should be added.

Experiment 5 (6.2 mole% water added)

Initial conversion of the $H_2/CO$ mixture: 86 mole%. Conversion of the $H_2CO$ mixture after 600 h: 86 mole%.

Of the above-mentioned experiments 1—5 only the numbers 2 and 5 are experiments according to the invention.

## Claims

1. A process for the preparation of an aromatic hydrocarbon mixture, in which a mixture of carbon monoxide and hydrogen is contacted with a mixture of two catalysts of which one is capable of catalyzing the conversion of an $H_2/CO$ mixture into acyclic oxygen-containing hydrocarbons and the other is a crystalline silicate, which silicate has the following properties after 1 hour's calcining in air at 500°C:

(a) an X-ray powder diffraction pattern showing, inter alia, the reflections given in Table A,

TABLE A

| Radiation: Cu-K$\alpha$ 2 $\theta$ | Wavelength 0.15418 nm relative intensity |
|---|---|
| 7.8—8.2 | S |
| 8.7—9.1 | M |
| 11.8—12.1 | W |
| 12.4—12.7 | W |
| 14.6—14.9 | W |
| 15.4—15.7 | W |
| 15.8—16.1 | W |
| 17.6—17.9 | W |
| 19.2—19.5 | W |
| 20.2—20.6 | W |
| 20.7—21.1 | W |
| 23.1—23.4 | VS |
| 23.8—24.1 | VS |
| 24.2—24.8 | S |
| 29.7—30.1 | M |

wherein the letters used have the following meanings:
VS=very strong; S=strong; M=moderate; W=weak; $\theta$=angle according to Bragg,

(b) after conversion of the silicate into the H-form and after evacuation at $2\times10^{-9}$ bar and 400°C for 16 hours and measured at a hydrocarbon pressure of $8\times10^{-2}$ bar and 100°C, the adsorption of n-hexane is at least 0.8 mmole/g, the adsorption of 2,2-dimethylbutane at least 0.5 mmole/g and the ratio

$$\frac{\text{adsorption of n-hexane}}{\text{adsorption of 2,2-dimethylbutane}} \text{ at least 1.5,}$$

(c) the composition, expressed in moles of the oxides, is as follows:

$$y(1.0\pm0.3)M_{2/n}O \cdot y(a\ Fe_2O_3 \cdot b\ Al_2O_3) \cdot SiO_2,$$

wherein M=H and alkali metal or alkaline-earth metal; n=the valency of M; $0<y\leqslant0.1$; $a\geqslant0$; $b\geqslant0$, and $a+b=1$, characterized in that to an $H_2/CO$ mixture with an $H_2/CO$ molar ratio between 0.25 and 0.75 an amount of water is added which—in mole%, based on the $H_2/CO$ mixture— is at least 2.5 and at most

$$\frac{3(V-R)}{(1+R)(1+V)}$$

wherein
R=the $H_2/CO$ molar ratio of the feed, and
V=the consumption ratio of the $H_2/CO$ mixture obtained under the conditions at which the above-mentioned process is carried out, but without water addition.

2. A process according to claim 1, characterized in that it is carried out at a temperature of 200—500°C, a pressure of 1—150 bar and a space velocity of 50—5000 l (NTP) gas/l catalyst/h.

3. A process according to any one of claims 1—2, characterized in that the catalyst mixture is built up of a catalyst A and a catalyst B, catalyst A being capable of converting an $H_2/CO$ mixture into substantially methanol and/or dimethyl ether, and catalyst B being the crystalline silicate.

4. A process according to claim 3, characterized in that as the A-catalyst a composition is used which contains zinc together with chromium.

5. A process according to claim 4, characterized in that in the A-catalyst the atomic percentage of zinc, based on the sum of zinc and chromium, is at least 60%.

6. A process according to any one of claims 3—5, characterized in that the catalyst mixture contains 1—5 parts by volume of catalyst A per part by volume of catalyst B.

7. A process according to claim 3, characterized in that as the A-catalyst a composition is used which contains zinc together with copper.

8. A process according to any one of claims 1—7, characterized in that the catalyst mixture contains a crystalline silicate whose average crystallite size is less than 3000 nm.

9. A process according to any one of claims 1—8, characterized in that the catalyst mixture contains a crystalline silicate of which, in the formula which gives the overall composition of the silicate, a=1.

10. Aromatic hydrocarbon mixtures which have been prepared using a process according to claim 9.

**Patentansprüche**

1. Verfahren zur Herstellung eines aromatischen Kohlenwasserstoffgemisches, in dem ein Gemisch aus Kohlenmonoxid und Wasserstoff mit einem Gemisch aus zwei Katalysatoren in Berührung gebracht wird, von denen der eine die Fähigkeit besitzt, die Umwandlung eiens $H_2/CO$-Gemisches in acylische sauerstoffhaltige Kohlenwasserstoffe zu katalysieren, und der andere ein kristallines Silikat ist, welches nach einstündiger Calcinierung in Luft bei 500°C die folgenden Eigenschaften aufweist:
(a) ein Röntgenbeugungspulverdiagramm zeigt unter anderem die in Tabelle A angegebenen Reflexionen.

TABELLE A

| Bestrahlung: Cu-K$\alpha$ 2 $\theta$ | Wellenlänge 0,15418 nm relative intensität |
|---|---|
| 7,8—8,2 | S |
| 8,7—9,1 | M |
| 11,8—12,1 | W |
| 12,4—12,7 | W |
| 14,6—14,9 | W |
| 15,4—15,7 | W |
| 15,8—16,1 | W |
| 17,6—17,9 | W |
| 19,2—19,5 | W |
| 20,2—20,6 | W |
| 20,7—21,1 | W |
| 23,1—23,4 | VS |
| 23,8—24,1 | VS |
| 24,2—24,8 | S |
| 29,7—30,1 | M |

wobei die verwendeten Buchstaben folgende Bedeutung haben:
VS=sehr stark; S=stark; M=mittel; W=schwach; $\theta$=Winkel gemäss dem Bragg'schen Gesetz.
(b) nach der Umwandlung des Silikats in die H-Form und nach dem Evakuieren bei $2\times10^{-9}$ bar und 400°C während 16 Stunden und Messen bei einem Kohlenwasserstoffdruck von $8\times10^{-2}$ bar und 100°C beträgt die Adsorption von n-Hexan mindestens 0,8 mMol/g, die Adsorption von 2,2-Dimethylbutan mindestens 0,5 mMol/g und das Verhältnis von

$$\frac{\text{Adsorption von n-Hexan}}{\text{Adsorption von 2,2-Dimethylbutan}} \text{ mindestens } 1{,}5,$$

(c) die Zusammensetzung, ausgedrückt in Mol der Oxide, ist wie folgt:

$$y(1{,}0\pm0{,}3)M_{2/n}O \cdot y(a\ Fe_2O_3 \cdot b\ Al_2O_3) \cdot SiO_2,$$

wobei M=Wasserstoff und Alkalimetall oder Erdalkalimetall, n die Wertigkeit von M, $0<y\leqslant0{,}1$, $a\geqslant0$, $b\geqslant0$ und $a+b=1$ ist, dadurch gekennzeichnet, dass einem $H_2/CO$-Gemisch mit einem molaren Verhältnis von $H_2$: CO zwischen 0,25 und 0,75 eine Wassermenge zugesetzt wird, welche—ausgedrückt in Mol-%, bezogen auf das $H_2/CO$-Gemisch—mindestens 2,5 und höchstens

$$\frac{3(V-R)}{(1+R)(1+V)} \text{ beträgt, wobei}$$

R=das molare Verhältnis von $H_2$: CO des Einsatzmaterials und
V=das Verbrauchsverhältnis des $H_2/CO$-Gemisches ist, welches unter den Bedingungen erhalten wird, unter denen das vorstehend erwähnte Verfahren durchgeführt wird, jedoch ohne Zugabe von Wasser.

2. Verfahren nach Anspruch 1, dadurch gekennzeichnet, dass dieses bei einer Temperatur von 200 bis 500°C, einem Druck von 1 bis 150 bar und einer Raumgeschwindigkeit von 50 bis 5000 l (NTD) Gas/l Katalysator/h durchgeführt wird.

3. Verfahren nach einem der Ansprüche 1 bis 2, dadurch gekennzeichnet, dass das Katalysatorgemisch aus einem Katalysator A und einem Katalysator B aufgebaut ist, wobei der Katalysator A die Fähigkeit besitzt, ein $H_2$/CO-Gemisch in im wesentlichen Methanol und/oder Dimethyläther umzuwandeln, und der Katalysator B das kristalline Silikat ist.

4. Verfahren nach Anspruch 3, dadurch gekennzeichnet, dass als A-Katalysator eine Zusammensetzung verwendet wird, die Zink zusammen mit Chrom enthält.

5. Verfahren nach Anspruch 4, dadurch gekennzeichnet, dass in dem A-Katalysator der Anteil an Zink, bezogen auf die Summe an Zink und Chrom, mindestens 60 Atom-% beträgt.

6. Verfahren nach irgendeinem der Ansprüche 3 bis 5, dadurch gekennzeichnet, dass das Katalysatorgemisch 1 bis 5 Volumenteile des Katalysators A je Volumenteil des Katalysators B enthält.

7. Verfahren nach Anspruch 3, dadurch gekennzeichnet, dass als A-Katalysator eine Zusammensetzung verwendet wird, die Zink zusammen mit Kupfer enthält.

8. Verfahren nach irgendeinem der Ansprüche 1 bis 7, dadurch gekennzeichnet, dass das Katalysatorgemisch ein kristallines Silikat enthält, dessen durchschnittliche Kristallitgrösse unter 3000 nm beträgt.

9. Verfahren nach irgendeinem der Ansprüche 1 bis 8, dadurch gekennzeichnet, dass das Katalysatorgemisch ein kristallines Silikat enthält, bei dem in der die Gesamtzusammensetzung des Silikats wiedergebenden Formel a=1 ist.

10. Aromatische Kohlenwasserstoffgemische, hergestellt unter Verwendung eines Verfahrens gemäss Anspruch 9.

**Revendications**

1. Un procédé pour la préparation d'un mélange d'hydrocarbures aromatiques, dans lequel un mélange d'oxyde de carbone de d'hydrogène est mis en contact avec un mélange de deux catalyseurs dont l'un est capable de catalyser la transformation d'un mélange $H_2$/CO en hydrocarbures acycliques contenant de l'oxygène et l'autre est un silicate cristallin, lequel silicate a les propriétés suivantes après calcination pendant 1 heure dans l'air à 500°C:

(a) il a un diagramme de diffraction des rayons X par la méthode des poudres présentant, entre autres, les réflexions indiquées dans le Tableau A,

TABLEAU A

| Radiation: Cu-K$\alpha$<br>$2\theta$ | Longueur d'onde 0,15418 nm<br>intensité relative |
|---|---|
| 7,8—8,2 | S |
| 8,7—9,1 | M |
| 11,8—12,1 | W |
| 12,4—12,7 | W |
| 14,6—14,9 | W |
| 15,4—15,7 | W |
| 15,8—16,1 | W |
| 17,6—17,9 | W |
| 19,2—19,5 | W |
| 20,2—20,6 | W |
| 20,7—21,1 | W |
| 23,1—23,4 | VS |
| 23,8—24,1 | VS |
| 24,2—24,8 | S |
| 29,7—30,1 | M |

où les lettres utilisées ont les significations suivantes:
VS=très forte; S=forte; M=modérée; W=faible; $\theta$=angle selon Bragg,

(b) après transformation du silicate en la forme H et après mise sous vide à $2\times10^{-9}$ bars et à 400°C pendant 16 heures et en effectuant les mesures à une pression d'hydrocarbures de $8\times10^{-2}$ barset à 100°C, l'adsorption de n-hexane est d'au moins 0,8 mmole/g, l'adsorption de 2,2-diméthylbutane d'au moins 0,5 mmole/g et le rapport

$$\frac{\text{adsorption de n-hexane}}{\text{adsorption de 2,2-diméthylbutane}} \text{ d'au moins 1,5,}$$

(c) la composition, exprimée en moles des oxydes, est la suivante:

$$y(1{,}0\pm0{,}3)M_{2/n}O \cdot y(a\ Fe_2O_3 \cdot b\ Al_2O_3) \cdot SiO_2,$$

où M=H et métal alcalin ou métalalcalino-terreux; n=la valence de M; $0<y\leqslant 0{,}1$; $a\geqslant 0$; $b\geqslant 0$, et $a+b=1$, caractérisé en ce qu'à un mélange $H_2/CO$ ayant un rapport molaire $H_2/CO$ compris entre 0,25 et 0,75, on ajoute une quantité d'eau qui, en moles %, par rapport au mélange $H_2/CO$, est au moins 2,5 et au maximum

$$\frac{3(V-R)}{(1+R)(1+V)}\text{, où}$$

R=le rapport molaire $H_2/CO$ de la charge et
V=le rapport de consommation du mélange $H_2/CO$ obtenu dans les conditions dans lesquelles le procédé mentionné ci-dessus est mis en oeuvre, mais sans addition d'eau.

2. Un procédé selon la revendication 1, caractérisé en ce qu'il est mis en oeuvre à une température de 200—500°C, une pression de 1—150 bars et une vitesse spatiale de 50—5000 litres (TPN) de gaz par litre de catalyseur et par heure.

3. Un procédé selon l'une quelconque des revendications 1 et 2, caractérisé en ce que le mélange de catalyseurs est constitué d'un catalyseur A et d'un catalyseur B, le catalyseur A étant capable de transformer un mélange $H_2/CO$ essentiellement en méthanol et/ou en oxyde de méthyle et le catalyseur B étant un silicate cristallin.

4. Un procédé selon la revendication 3, caractérisé en ce que comme catalyseur A on utilise une composition qui contient du zinc en même temps que du chrome.

5. Un procédé selon la revendication 4, caractérisé en ce que dans le catalyseur A la pourcentage atomique de zinc, par rapport à la somme de zinc et de chrome, est d'au moins 60%.

6. Un procédé selon l'une quelconque des revendications 3 à 5, caractérisé en ce que le mélange de catalyseurs contient de 1 à 5 parties en volume de catalyseur A par partie en volume de catalyseur B.

7. Un procédé selon la revendication 3, caractérisé en ce que comme catalyseur A on utilise une composition qui contient du zinc en même temps que du cuivre.

8. Un procédé selon l'une quelconque des revendicatons 1 à 7, caractérisé en ce que le mélange de catalyseurs contient un silicate cristallin dont la grosseur moyenne des cristallites est inférieure à 3000 nm.

9. Un procédé selon l'une quelconque des revendications 1 à 8, caractérisé en ce que le mélange de catalyseurs contient un silicate cristallin pour lequel, dans la formule qui donne la composition d'ensemble du silicate, a=1.

10. Mélanges d'hydrocarbures aromatiques qui ont été préparés en utilisant un procédé selon la revendication 9.